(19) Europäisches Patentamt
European Patent Office
Office européen des brevets

(11) Numéro de publication: 0 179 076
B1

(12) # FASCICULE DE BREVET EUROPEEN

(45) Date de publication du fascicule du brevet: **26.10.88**

(51) Int. Cl.[4]: **C 07 D 233/76,** A 61 K 31/415

(21) Numéro de dépôt: **85901329.4**

(22) Date de dépôt: **16.04.85**

(86) Numéro de dépôt international: **PCT/BE 85/00007**

(87) Numéro de publication internationale: **WO 85/05103 (21.11.85** Gazette **85/25)**

(54) **DERIVES SULFURES DE L'HYDANTOINE ET COMPOSITIONS PHARMACEUTIQUES LES CONTENANT.**

(30) Priorité: **27.04.84 BE 212840**

(43) Date de publication de la demande: **30.04.86 Bulletin 86/18**

(45) Mention de la délivrance du brevet: **26.10.88 Bulletin 88/43**

(84) Etats contractants désignés: **AT BE CH DE FR GB IT LI LU NL SE**

(56) Documents cités:
**DE - A - 2 825 245**

**European Journal of Medicinal Chemistry - Chimica Therapeutica, vol. XV, no. 6, November-December 1980, Châtenay-Malabry(FR), J.H. Poupaert et al.: "Search for pro-drug forms of D-Penicillamine: Synthesis and metabolism of D-Pen-Hydantoin", pp. 511-14**

(73) Titulaire: **Région Wallonne représentée par l'Exécutif Régional Wallon, 11, Boulevard de l'Empereur, B-1000 Bruxelles (BE)**

(72) Inventeur: **DUMONT, Pierre, 13, avenue Moine Olbert, B-5800 Gembloux (BE)**
Inventeur: **POUPAERT, Jacques, 22, rue du Palier, B-1348 Louvain-la-Neuve (BE)**

(74) Mandataire: **Blanchart, André, MINISTERE DE LA REGION WALLONE Direction d'Administration de l'Energie et des Technologies nouvelles Service des Technologies nouvelles Avenue Prince de Liège, 7, B-5100 Namur (BE)**

EP 0 179 076 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention a pour objet de nouveaux dérivés sulfurés de l'hydantoïne utilisés dans le traitement de fond de l'arthrite rhumatoïde ou polyarthrite chronique évolutive. Ces substances visent à corriger la réponse inflammatoire chronique qui caractérise cette infection et se distinguent de la sorte très nettement des antiinflammatoires non stéroïdiens, qui atténuent la réaction inflammatoire aiguë durant les épisodes d'exacerbation mais sans enrayer pour autant le décours de cette maladie.

Il est connu pour le traitement de telles maladies d'utiliser en clinique les antimalariques, les complexes d'or et la D-pénicillamine.

Cette dernière molécule possède une activité bien établie mais est responsable de nombreux effets secondaires qui en limitent l'emploi et sont à l'origine d'abandon en cours de traitement. Suivant DE-A-28 25 245, les dérivés de l'hydantoïne substitués en position 5 sont connus mais n'ont pas été utilisés en thérapeutique.

Suivant Europ. J. Med. Chem. – Chimica Therapeutica XV (1980) pages 511–514, il est connu d'utiliser le (méthyl-1-thiol-1-éthyl)-5 hydantoïne comme précurseur de D- pénicillamine. Toutefois, ce composé n'a pas été utilisé thérapeutique du fait que le métabolisme de l'hydantoïne ne correspond pas à celui de la D-pénicillamine. La présente invention a pour objet de nouveaux produits pharmaceutiques à base de dérivés sulfurés de l'hydantoïne et leurs utilisations dans des compositions pharmaceutiques. De tels composés possèdent des caractéristiques structurales telles qu'elles assurent un devenir différent de celui de la D-pénécillamine tout en maintenant une activité comparable, réalisant ainsi la possibilité d'atteindre une moindre toxicité et un meilleur indice thérapeutique.

Les nouveaux dérivés sulfurés de l'hydantoïne de l'invention sont constitués d'une structure répondant à la formule générale (I)

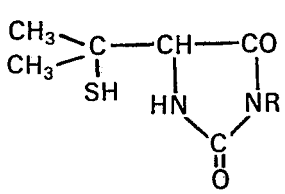

I

dans laquelle R représente un atome d'hydrogène, un groupe alkyle contenant de 1 à 6 atomes de carbone, un groupe cycloalkyle contenant de 3 à 7 atomes de carbone, ou un groupe aryle de 6 atomes de carbone. Les nouveaux dérivés sulfurés de l'hydantoïne comprennent également leurs sels physiologiquement acceptables, leurs isomères optiques, leurs mélanges et leurs racémiques. Par sels physiologiquement acceptables, on comprend les sels obtenus par l'action de composés alcalins ou alcalino-terreux suivant les procédés bien connus, ou tout autre procédé décrit dans la littérature.

Les dérivés sulfurés de l'hydantoïne revendiqués possèdent un carbone asymétrique en position 5, qui leur confère une activité optique. Les isomères optiques (énantiomères) qui recouvrent les composés de l'invention sont désignés classiquement par les symboles (R) et (S), ou encore par L et D, 1 et d, (+) ou (−), ou par des combinaisons de ces symboles.

Lorsqu'on a pas attribué de désignation spécifique aux composés de l'invention, ces composés désignent tant les énantiomères individuels, que leurs mélanges ou leurs racémiques.

Les compositions pharmaceutiques de la présente invention peuvent comprendre un seul des dérivés actifs de l'hydantoïne ou des mélanges de plusieurs de ces dérivés, pris dans le sens le plus large, et comprenant notamment leurs sels et leurs isomères optiques, leurs mélanges et leurs racémiques. Les compositions pharmaceutiques résultent d'une mise sous forme de dosage approprié, éventuellement avec des véhicules et/ou excipients solides ou liquides, inertes, non toxiques, utilisés en pharmacie gelénique. Le choix de présentation des compositions sera fait en fonction de l'administration orale, parentérale ou tout autre voie. Ainsi, on donnera à la préparation galénique la forme de comprimés, de cachets, de capsules, de dragées, de solutions buvables ou injectables, ou de suppositoires.

La dose unitaire utilisée correspondra à une dose pharmacologiquement active établie suivant des procédés bien connus de l'homme de l'art.

Parmi les nouveaux dérivés sulfurés de l'hydantoïne utilisés comme composés actifs des compositions pharmaceutiques, le dérivé le plus simple est obtenu dans le cas de R = H, dans la formule générale (I). Dans ce cas, le composé est la (méthyl-1 thiol-1 éthyl)-5 hydantoïne.

Le substituant R peut être un groupe alkyle de 1 à 6 atomes de carbone (méthyle, éthyle, n-propyle, isopropyle, n-butyle, n-pentyle, n-hexyle,). De préférence, on choisit le groupe méthyle ou éthyle. Dans ces cas, les dérivés de l'hydantoïne sont la méthyl-3 (méthyl-1 thiol-1 éthyl)-5 hydantoïne et l'éthyl-3 (méthyl-1 thiol-1 éthyl)-5 hydantoïne. Le substituant R peut être un groupe cycloalkyle contenant de 3 à 7 atomes de carbone choisi parmi le cyclopropyle, le cyclobutyle, le cyclopentyle, le cyclohexyle et le cycloheptyle. On choisit de préférence le cyclohexyle. Dans ce cas, le composé est la cyclohexyl-3 (méthyl-1 thiol-1 éthyl)-5 hydantoïne.

Le substituant R peut être également un groupe aryle choisi de 6 atomes de carbone, de préférence le groupe phényle. Dans ce cas, le composé en résultant est la phényl -3 (méthyl-1 thiol-1 éthyl)-5 hydantoïne.

La présente invention a également pour objet les compositions pharmaceutiques utilisées comme immunomodulateur et/ou dans le traitement de l'arthrite rhumatoïde, contenant en une quantité thérapeutiquement efficace les nouveaux dérivés sulfurés de l'hydantoïne répondant à la formule générale (I). Les produits pharmaceutiques revendiqués et les compositions pharmaceutiques contenant une dose thérapeutiquement efficace de ces produits manifestent une activité pharmacologique comparable à celle de la D-pénicillamine. Il est surprenant de constater une telle activité. En effet, malgré leur structure relativement éloignée de celle de la D-pénécillamine, les composés revendiqués manifestent une activité élevée dans le test d'hypersensibilité retardée au chlorure de picryle, qui est considéré actuellement comme un des tests les plus représentatifs de l'activité thérapeutique des substances destinées au traitement de l'arthrite rhumatoïde. Les produits et compositions pharmaceutiques de l'invention ne représentent pas d'activité anticonvulsivante fréquemment rencontrée parmi les dérivés du noyau hydantoïne et ceci par application du test à l'électrochoc. Enfin leur toxicité aiguë, estimée par la mesure de DL50 sur rongeur est inférieure à celle de la D-pénicillamine. Le procédé de synthèse fait appel à une méthode connue (réaction de Dakin), mais adaptée aux exigences de stabilité.

La préparation des substances actives suivant l'invention est décrite de manière plus détaillée à l'aide des exemples suivants sans être pour autant limité à ces derniers.

Exemple 1

Préparation de (S)-(méthyl-1 thiol-1 éthyl)-5 hydantoïne.

Sous un courant continu d'azote, on chauffe à 80 °C durant 30 min. une solution de 30 g de D-pénicillamine et 30 g de cyanate de potassium dans 100 ml d'eau distillée et dégazée. Le mélange réactionnel est ensuite refroidi dans la glace et acidifié au moyen d'acide chlorhydrique 6N. Le précipité ainsi obtenu est essoré, lavé à l'eau distillée et mis en suspension dans 600 ml d'acide chlorhydrique 3N. On chauffe à 95° durant 40 min. Après refroidissement dans la glace, on essore un produit cristallin, qui est recristallisé dans le méthanol : eau. Le produit résultant, qui est la (S) – (méthyl-1 thiol-1 éthyl)-5 hydantoïne, a un point de fusion de 247–248 °C et présente une activité optique $(\alpha)_{546}$ + 107° (concentration de 0,1 g dans 100 ml de méthanol).

Exemple 2

Préparation de (S)-méthyl-3 (méthyl-1 thiol-1 éthyl)-5 hydantoïne.

A une solution de 2 g de D-pénicillamine et 0,536 g de NaOH dans 20 ml d'eau distillée dégazée et refroidie à 4 °C, on ajoute sous agitation en une fois 1 ml de pyridine et goutte à goutte 0,840 g d'isocyanate de méthyle.

L'opération est menée sous courant d'azote. Le mélange réactionnel est ramené progressivement à la température ordinaire. Après 24 h, on extrait deux fois par 20 ml de benzène ou de toluène. La phase aqueuse est additionnée de 20 ml d'acide chlorhydrique 6N et chauffée à 95 °C durant 120 min. Par refroidissement à la température ordinaire, on obtient 1,6 g d'un précipité qui est essoré, lavé à l'eau distillée et séché sous vide. Après recristallisation dans le méthanol : eau, on obtient la (S) méthyl – 3 (méthyl-1-thiol-1 éthyl)-5 hydantoïne dont le point de fusion est de 157–158 °C et le pouvoir optique $(\alpha)_{546}$ + 104° (concentration de 0,1 g dans 100 ml de méthanol).

Exemple 3

Préparation de (R)-éthyl – 3 (méthyl-1 thiol-1 éthyl) – 5 hydantoïne.

On procède comme dans l'exemple 2, mais en substituant l'isocyanate de méthyle par une quantité équivalente d'isocyanate d'éthyle.

Le produit obtenu est caractérisé par un point de fusion de 116–118 °C et une activité optique $(\alpha)_{546}$ – 109° (concentration de 0,1 g dans 100 ml de méthanol).

Exemple 4

Préparation de (R,S) – cyclohexyl-3 (méthyl-1 thiol-1 éthyl) -5 hydantoïne.

On procède comme pour l'exemple 2, mais en substituant l'isocyanate de méthyle par une quantité équivalente d'isocyanate de cyclohexyle. Le produit obtenu est caractérisé par un point de fusion de 156–158 °C.

Exemple 5

Préparation de (R,S)-phényl-3 (méthyl-1 thiol-1 éthyl)-5 hydantoïne.

On procède comme dans l'exemple 2, mais en substituant l'isocyanate de méthyle par une quantité équivalente d'isocyanate de phényle.

Le produit obtenu est caractérisé par un point de fusion de 109–110 °C.

Exemple 6

Composition pharmaceutique à usage oral

50 mg d'un des composés revendiqués est dispersé dans 200 mg de lactose et mis dans une gélule de format approprié. Pour étudier l'activité des composés revendiqués quant à leurs propriétés immunomodulatrices, et plus particulièrement quant à leurs effets sur l'immunité cellulaire, on les a soumis au test d'hypersensibilité retardée au chlorure de picryle. Ce test est effectué sur souris mâles de la souche NMRI de poids moyen 30 g. Par produit testé, 10 animaux sont utilisés. Dix animaux non-traités au chlorure de picryle constituent un lot contrôle et dix autres un lot témoin (animaux traités au chlorure de picryle mais n'ayent reçu aucun produit revendiqué). Au jour 1 la peau de l'abdomen de la souris est mis à nu. On étend 0,1 ml d'une solution à 5% dans l'acétone de chlorure de picryle. Au jour 2 on administre par os et par 10 g de poids corporel 0,1 ml d'une suspension de gomme adragante à 1% dans l'eau distillée contenant le produit à tester. La dose des différents produits est de 0,335 mole par kg. Le lot contrôle reçoit le même volume de suspension de gomme adragante. Au jour 3 les deux faces de l'oreille droite sont badigeonnées avec 5 µl d'une solution de chlorure de picryle à 1% dans un mélange acétone – phthalate de butyle 50 : 50 (challenge). Ces animaux reçoivent par gavage le même produit à tester qu'au jour 2, deux heures avant et deux heures après le challenge. Au jour 4,

les animaux sont sacrifiés par inhalation d'éther. L'épaisseur du pavillon de l'oreille est mesurée chez chacun des animaux. L'activité se traduit par une réduction du gonflement du pavillon, comparativement au lot témoin. A titre d'exemple, nous présentons l'activité des composés : (S) – (méthyl-1 thiol-1 éthyl) – 5 hydantoïne(composé 1) et (S) – méthyl-3 (méthyl-1 thiol-1 éthyl)-5 hydantoïne (composé 2) comparativement à la D-pénicillamine.

Tableau I
% de réduction du gonflement du pavillon de l'oreille sur souris NMRI.

| lot traité par | % |
|---|---|
| composé 1 | 80 |
| composé 2 | 70 |
| D-pénicillamine | 75 |

Il ressort nettement de ces essais que les produits sulfurés revendiqués dans la présente invention manifestent une activité pharmacologique de type D-pénicillamine, et ceci bien que leur structure soit sensiblement éloignée de celle de ce dernier composé.

Les composés revendiqués ne présentent plus la structure d'acide aminé et par conséquent présente une toxicité chronique moindre et un meilleur indice thérapeutique dans le traitement de fond.

## Revendications

1. Produits pharmaceutiques à base de dérivés sulfurés de l'hydantoïne, caractérisés par la formule générale (I)

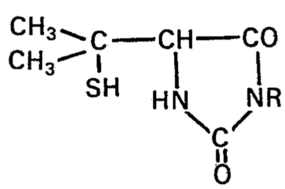

I

dans laquelle R représente un atome d'hydrogène, un groupe alkyle contenant de 1 à 6 atomes de carbone, un groupe cycloalkyle contenant de 3 à 7 atomes de carbone, ou un groupe aryle de 6 atomes de carbone, ainsi que leurs sels physiologiquement acceptables, leurs isomères optiques, leurs mélanges et leurs racémiques.

2. Produits pharmaceutiques à base de dérivés sulfurés de l'hydantoïne suivant la revendication 1, caractérisés en ce que le substituant R est choisi parmi les groupes méthyle, éthyle, cyclohexyle ou phényle.

3. Produits pharmaceutiques à base de dérivés sulfurés de l'hydantoïne suivant les revendications 1 et 2, caractérisés en ce qu'ils comprennent la (méthyl-1 thiol-1 éthyl)-5 hydantoïne, la méthyl-3 (méthyl-1 thiol-1 éthyl)-5 hydantoïne, l'éthyl-3 (méthyl-1 thiol-1 éthyl)-5 hydantoïne, la cyclohexyl-3 (méthyl-1 thiol-1 éthyl) -5 hydantoïne, la phényl -3 (méthyl-1 thiol-1 éthyl) - 5 hydantoïne, et leurs isomères optiques, leurs mélanges et leurs racémiques.

4. Compositions pharmaceutiques utilisées comme immunomodulateur et/ou pour le traitement de l'arthrite rhumatoïde, caractérisées en ce qu'elles comprennent en une quantité thérapeutiquement efficace les produits pharmaceutiques faisant l'objet des revendications 1 à 3.

## Patentansprüche

1. Pharmazeutische Produkte auf der Basis von sulfurierten Derivaten des Hydantoins, gekennzeichnet durch die allgemeine Formel (I)

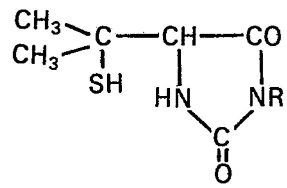

I

in welcher R ein Wasserstoffatom, eine Alkylgruppe mit 1 bis 6 Kohlenstoffatomen, eine Cycloalkylgruppe mit 3 bis 7 Kohlenstoffatomen oder eine Arylgruppe mit 6 Kohlenstoffatomen bedeutet, sowie deren physiologisch annehmbaren Salze, deren optischen Isomeren, deren Mischungen und Racemate.

2. Pharmazeutische Produkte auf der Basis sulfurierter Derivate des Hydantoins gemäss Anspruch 1, dadurch gekennzeichnet, dass der Substituent R ausgewählt ist unter den Gruppen Methyl, Ethyl, Cyclohexyl oder Phenyl.

3. Pharmazeutische Produkte auf der Basis von sulfurierten Derivaten des Hydantoins gemäss Ansprüchen 1 und 2, dadurch gekennzeichnet, dass sie umfassen das (Methyl-1 thiol-1 ethyl)-5 hydantoin, das Methyl -3 (methyl-1 thiol-1 ethyl) -5 hydantoin, das Ethyl-3 (methyl-1 thiol-1 ethyl)-5 hydantoin, das Cyclohexyl-3 (methyl-1 thiol-1 ethyl) -5 hydantoin, das Phenyl -3 (methyl-1 thiol-1 ethyl) - 5 hydantoin und deren optischen Isomere, Mischungen und Racemate.

4. Pharmazeutische Zusammensetzungen zur Verwendung als Immunmodulatoren und/oder für die Behandlung der rheumatoiden Arthritis, dadurch gekennzeichnet, dass sie eine therapeutisch wirksame Menge der pharmazeutischen Produkte gemäss einem der Ansprüche 1 bis 3 enthalten.

## Claims

1. Pharmaceutical products based on sulphurated derivatives of hydantoin, characterised by the general formula (I)

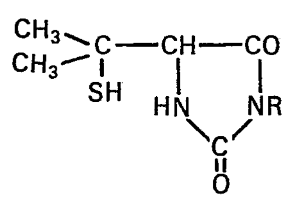

I

in which R represents a hydrogen atom, an alkyl group containing from 1 to 6 carbon atoms, a cycloalkyl group containing from 3 to 7 carbon atoms or an aryl group with 6 carbon atoms, and also physiologically acceptable salts, optical isomers, mixtures and racemates thereof.

2. Pharmaceutical products based on sulphurated derivatives of hydantoin according to claim 1, characterised in that the substituent R is chosen from methyl, ethyl, cyclohexyl or phenyl groups.

3. Pharmaceutical products based on sulphurated derivatives of hydantoin according to claims 1 and 2, characterised in that they comprise 5-(1-methyl-1-thiol ethyl) hydantoin, 3-methyl-5-(1-methyl-1-thiol ethyl) hydantoin, 3-ethyl-5-(1-methyl-1-thiol ethyl) hydantoin, 3-cyclohexyl-5-(1-methyl-1-thiol ethyl) hydantoin, 3-phenyl-5-(1-methyl-1-thiol ethyl) hydantoin, and optical isomers, mixtures and racemates thereof.

4. Pharmaceutical compositions used as an immunomodulator and/or for the treatment of rheumatoid arthritis, characterised in that they comprise a therapeutically effective quantity of the pharmaceutical products forming the subject-matter of claims 1 to 3.